# EUROPÄISCHE PATENTSCHRIFT

(11) **EP 0 648 108 B1**
(45) Veröffentlichungstag und Bekanntmachung des Hinweises auf die Patenterteilung: **20.11.1996**
(21) Anmeldenummer: 93912643.9
(22) Anmeldetag: 30.06.1993
(51) Int. Cl.: A61K 7/16, A61K 7/26

(54) **PRÄPARAT ZUR PROPHYLAKTISCHEN UND THERAPEUTISCHEN BEHANDLUNG VON KARIES**
PROPHYLACTIC AND THERAPEUTICAL PREPARATION AGAINST CARIES
COMPOSITION UTILISEE POUR LA PROPHYLAXIE ET LA THERAPIE DES CARIES

(30) Priorität: 30.06.1992 DE 4221054
(43) Veröffentlichungstag der Anmeldung: 19.04.1995
(73) Patentinhaber: WIEDEMANN, Wolfgang, D-97204 Höchberg (DE)
(72) Erfinder: WIEDEMANN, Wolfgang, D-97204 Höchberg (DE)
(74) Vertreter: Pöhner, Wilfried Anton, Dr.
(86) Internationale Anmeldenummer: DE9300579
(87) Internationale Veröffentlichungsnummer: WO9400101

(56) Entgegenhaltungen:
- EP-A- 0 091 611
- EP-A- 0 174 704
- EP-A- 0 344 701
- BE-A- 731 578
- DE-A- 729 572
- FR-A- 2 287 234
- GB-A- 2 102 289
- US-A- 4 080 440

## Beschreibung

Die Erfindung betrifft ein Präparat zur prophylaktischen und therapeutischen Behandlung von Karies in Form einer Kaumasse, von Kaubonbons, Lutschbonbons, Zahnpasta, Mundspray oder anderen Mundpflegemitteln, mit Calzium und Phosphat, die in gelöster oder leicht löslicher Form vorliegen, sowie einem Protonenspender.

Aus der US-A-4,080,440 ist ein Präparat zur Behandlung von Karies bekannt, das unmittelbar vor der oralen Anwendung aus einer Lösung mit Calzium und aus einer Lösung mit Phosphat hergestellt wird. Es entsteht dabei eine übersättigte, metastabile Lösung mit einem niedrigen pH-Wert, der von 2,5 bis 4 reicht. Das Calziumphosphat fällt innerhalb kurzer Zeit nach dem Einführen des Präparats in den Mund aus und lagert sich an der Zahnoberfläche ab. Nachteilig wirkt sich aus, daß der Zahn damit lediglich an der Oberfläche versiegelt wird; eine tiefgehende Remineralisierung des Zahnes findet nicht statt. Ein weiterer Nachteil ist, daß das Präparat erst kurz vor der Anwendung herstellbar ist, weil sonst das Calziumphosphat vor der Einführung in den Mund ausfällt. Somit ist die getrennte Aufbewahrung zweier Lösungen notwendig.

Davon ausgehend hat sich die Erfindung die Schaffung eines Präparates zur Kariesbehandlung zur Aufgabe gestellt, das eine vollständige und tiefe Remineralisierung eines Zahnes ermöglicht.

Zur Lösung dieser Aufgabe wird vorgeschlagen, daS spätestens bei der Anwendung in der Mundhöhle Calzium und Phosphat in eine gesättigte und stabile Lösung übergehen und der Säureanteil derart gewählt ist, daS sich der pH-Wert auf einen Wert zwischen 4 und 4,5 einstellt. Das Präparat wird oral angewendet, z.B. in Form eines Lutsch- oder Kaubonbons oder einer Kaumasse. Auch Zahnpasta, Mundwasser, Mundspray oder andere Präparate für die tägliche Zahn- und Mundpflege kommen als Vehikel in Betracht.

Die Erfindung geht aus von der Erkenntnis, daß der Prozeß der Kariesentstehung umkehrbar ist. Sowohl experimentelle Untersuchungen als auch ein mathematisches Diffusions-Reaktionsmodell haben ergeben, daß den nicht-stationären Randbedingungen für die Mineralaustauschvorgänge an den Zähnen besondere Bedeutung zukommt: Nur adäquat variierende Konzentrationen von gelöstem Mineral (im wesentlichen Calzium und Phosphat) und der pH-Wert sowie Hilfsstoffe wie Fluorid im Mundhöhlenmillieu, welches die Läsion umspült, ermöglichen eine vollständige und tiefe Remineralisation der Läsion. Die gleichen Vorgänge spielen eine bedeutsame Rolle bei der prophylaktischen Sättigung der Zahnhartsubstanzen mit Mineralien.

Der beschriebene Mineralisationsprozeß wird ganz wesentlich von physikochemischen Größen wie der Ab-scheidungsrate von Mineral aus der gelösten Phase, der Sättigungskonzentration beim jeweiligen pH-Wert oder den Diffusionskonstanten der verschiedenen gelösten Komponenten bestimmt. Diese Parameter sowie die Substratoberfläche, also die aktive Oberfläche im Schmelz, haben Einfluß auf Menge, Art und Struktur des abgeschiedenen Minerals.

Diese zunächst in vitro beobachteten Vorgänge funktionieren auch in vivo.

Die Verwendung dieser Erkenntnisse zur Entwicklung eines Kariesprophylaktikums oder -therapeutikums ist ganz wesentlich von der Möglichkeit bestimmt, die optimalen Verhältnisse in der Mundhöhle bzw. direkt am Zahn zu erzeugen. Hierzu zählt auch die Schaffung von für die Remineralisation optimalen Bedingungen, die im saueren Bereich liegen. Die Beigabe eines Protonenspenders dient der Ausbildung eines (zunächst von neutralen Verhältnissen ausgehenden) niederen pH-Werts. Erst dann erfolgt eine Dissoziation der Ca- und Phosphatverbindungen.

Für einen Lutschbonbon können als Grundmasse Zucker oder Zuckeraustauschstoffe wie Xylit, Sorbit oder Isomalt Verwendung finden. Als Grundmasse kommen weiterhin in Betracht Zuckeraustauschstoffe und/oder Gelatine und/oder Gummiarabicum und/oder Kaumasse.

Bei der Formulierung des Rezeptes für eine Applikation muß die Pufferwirkung des Phosphatrestes sowie die des Speichels berücksichtigt werden. Einen wichtigen Gesichtspunkt für die Zusammensetzung stellt auch die Speichel-Clearance dar, d.h. die Menge an zugeführten Agentien, die durch den Speichel verdünnt und verschluckt wird. Dieser Anteil wird stark von der Applikationsform (Lutschbonbon, Kaubonbon, Zahnpasta ...) beeinflußt. Bei dem für eine gesteuerte Remineralisation notwendigen Ausgangs-pH-Wert von pH=4-4,5 liegt ein Teil des Phosphates als H₂PO₄-Ion, also nur einfach dissoziiert in Lösung vor. Die undissoziierten, an das Phosphat gebundenen Protonen müssen zusätzlich zugegeben werden. Das kann z.B. in Form von Citrat oder einer anderen in der Süßwarenherstellung zur Aromatisierung verwendeten Säure (z.B. Milchsäure, Apfelsäure, Weinsäure, allg. Fruchtsäure) geschehen. Auch die Verwendung von Calziumchlorid als Calziumspender wäre denkbar.

Um die Clearance der aus der Vorrichtung stammenden Stoffe zu verzögern und somit die Konzentrationsprofile zu steuern, können den Vorrichtungen, insbesondere der Zahnpasta, dem Mundwasser und dem Mundspray auch Stoffe beigegeben werden, die einerseits Calzium und/oder Phosphat in größerer Menge physikalisch binden, andererseits aber selbst an Zahnoberfläche und/oder Mundhöhlenschleimhaut adsorbiert werden. Solche Stoffe sind meist organischer Natur und bipolar und werden in der Kariesthereapie als Fluoridüberträger verwandt wie z.B. Aminfluorid. Auch Stoffe aus der Klasse der Chlorhexidin-di-Gluconate und der quarternären Ammoniumbasen werden für ähnliche Zwecke, z.B. zur Verlängerung der desinfizierenden Wirkung verwendet (z.B. bei Chlorhexidin und Benzoxoniumchlorid).

Aber auch Zusätze mit höherer Viskosität, die sich auf dem Zahn und der Schleimhaut auflagern und Calzium und Phosphat adsorbiert enthalten können, kommen zur Verzögerung der Clearance in Frage. Die Natur bildet solche Stoffe z.B. in der Zahnplaque in Form von extrazellulären Polysacchariden. Danach kann ein solcher Zusatz z.B. ein Stoff aus der Klasse der Polyglucane sein.

In saurem Milieu fällt Ca-PO4 oft nicht in solider Form oder der energetisch günstigsten Form, dem Hydroxylapatit, aus. Darum sind der Formulierung Begleitstoffe zuzuschlagen, die die solide Hydroxylapatit-Bildung katalysieren und die fraktale Abscheidung vermeiden. Als solcher Stoff ist z.B. Fluorid bekannt.

Die Ausführungen zeigen, daß sich für die Zusammensetzung des Präparates je nach Anwendungsform unterschiedliche Formulierungen ergeben:
a) für Lutschbonbons oder Kaubonbons mit einer mittlerer Verweilzeit von 5-8 min im Mund kann der oben beschriebene Mechanismus unter Berücksichtigung des Speichelflusses durch die folgende Zusammensetzung realisiert werden. Hierbei ist auch der ständige Verlust an mineralischen Bestandteilen durch Verschlucken der Lösungen berücksichtigt:
   Pro kg Bonbonmasse müssen 200 bis 800 mmol, vorzugsweise 300 bis 600 mmol, insbesondere 400 bis 500 mmol Calzium bzw. Calziumverbindungen zugegeben werden, das entspricht etwa 0,9 bis 3,6 Gewichtsprozent bzw. 1,35 bis 2,7 Gewichtsprozent bzw. 1,8 bis 2,25 Gewichtsprozent Calzium(-Verbindungen).
   Wegen des im Speichel vorhandenen hohen Phosphatgehaltes (Gehalt an Phosphat-Ionen) kann die Zufuhr von Phosphat(-Ionen) durch die beschriebene Vorrichtung zwischen 50 und 400 mmol, insbesondere 100 bis 300 mmol begrenzt werden. In Gewichtsprozent ausgedrückt sind dies 0,47 bis 3,73 bzw. 0,94 bis 2,82 Prozent PO₄.
   Ein Teil der für die Ansäuerung notwendigen Protonen wird von Phosphat(-Ionen) abgepuffert. Dadurch sowie durch die unvollständige Dissoziation der verschiedenen Fruchtsäuren geht er zur Ansäuerung des Mundhöhlenmilieus verloren. Die Zugabe an Säure richtet sich nach diesen Verhältnissen. Zur Ansäuerung des Mundhöhlenmilieus sind 290 mmol H+ pro kg Bonbonmasse notwendig (dieser Wert beruht auf Messungen, die im Mund durchgeführt wurden).
   Unter Berücksichtigung der o.a. Verhältnisse ergibt sich bei der Verwendung eines Calzium-Fruchtsäuresalzes als Calziumspender ein notwendiger Gehalt von 400 bis 1500 mval Fruchtsäure.
   Umgerechnet auf die dreibasische Zitronensäure ergibt sich je nach Dissoziationsgrad ein Gehalt von ca. 2,8 bis 11 Gewichtsprozent Citrat. Zur Katalyse der soliden Hydroxylapatit-Abscheidung sollte der Bonbon noch 0,7 bis 2,4 mmol Fluorid/kg Bonbon enthalten.
b) Für die Anwendung als Zahnpasta muß unter Berücksichtigung des Verdünnungseffektes durch den Speichel und dessen Pufferkapazität eine Formulierung in den folgenden Grenzen in Betracht gezogen werden:
   160 bis 660 mmol Ca, vorzugsweise 240 bis 500 mmol, insbesondere 320 bis 400 mmol Ca und 40 bis 330 mmol, vorzugsweise 80 bis 250 mmol PO4. An Säure benötigt man zwischen 300 und 1200 mval. Alle Angaben beziehen sich auf 1 Kg Zahnpasta.
   Ein Teil des Calzium und Phosphates kann auch, wie beschrieben, in einen adsorbierbaren Trägerstoff gegeben werden.
   Die Applikationsform als Zahnpasta hat, wie auch die nachfolgend beschriebene, den großen Vorteil, daß sie im Mund gleichmäßig verteilt wird und sehr homogene Verhältnisse erzeugt. Dem Trägerstoff kommt hierbei wegen der relativ kurzen Verweilzeit der eigentlichen Zahnpasta im Mund eine besondere Bedeutung zu.
c) Die Konzentrationen im Mundwasser liegen wegen der relativ großen Flüssigkeitsmengen, die beim Spülen aufgenommen werden, niedriger als bei den anderen beiden Formulierungen. Um die für die Härtung des Zahnschmelzes günstigsten Bedingungen in der Mundhöhle herzustellen, kann eine Mundspüllösung etwa wie folgt zusammengesetzt sein:
   5 bis 22 mmol Ca, vorzugsweise 8 bis 16 mmol, insbesondere 10 bis 15 mmol Ca und 1 bis 10, vorzugsweise 3 bis 8 mmol PO₄ sowie 10 bis 40 mval Säure/kg Mundwasser. Diese Werte beziehen sich auf eine gebrauchsfertige Lösung mit 30 ml/Applikation. Die Formulierungen in den Beispielen b und c sind ebefalls durch geringe Fluoridgaben zu ergänzen. Alle diese Formulierungen erzeugen während einer bestimmten Zeit in der Mundhöhle ein Milieu, in welchem der Zahnschmelz bei abgesenktem pH-Wert mit gelösten Mineralien aus einer gesättigten Lösung getränkt wird. Durch den Verbrauch des sauren Bonbons oder das Auswaschen der Calzium und Phosphat adsorbierenden Trägersubstanz in Zahnpasten oder Mundwässern kommt es zu einem Anstieg des pH-Wertes an der Oberfläche und einem Konzentrationsgefälle zwischen der Oberfläche des Schmelzes und seinem Inneren. Dadurch ist ein Diffusionsstrom von Protonen, Calzium- und Phosphat-Ionen in Richtung Außenmilieu bedingt. Wegen der gegenüber Calzium- und Phosphat-Ionen größeren Beweglichkeit der Protonen kommt es im Inneren des Schmelzes zu einem schnelleren Wiederanstieg des pH-Wertes. Dadurch werden die Calzium- und Phosphat-Ionen unter der Schmelzoberfläche zum Niederschlag gezwungen und scheiden sich als solides Material ab.

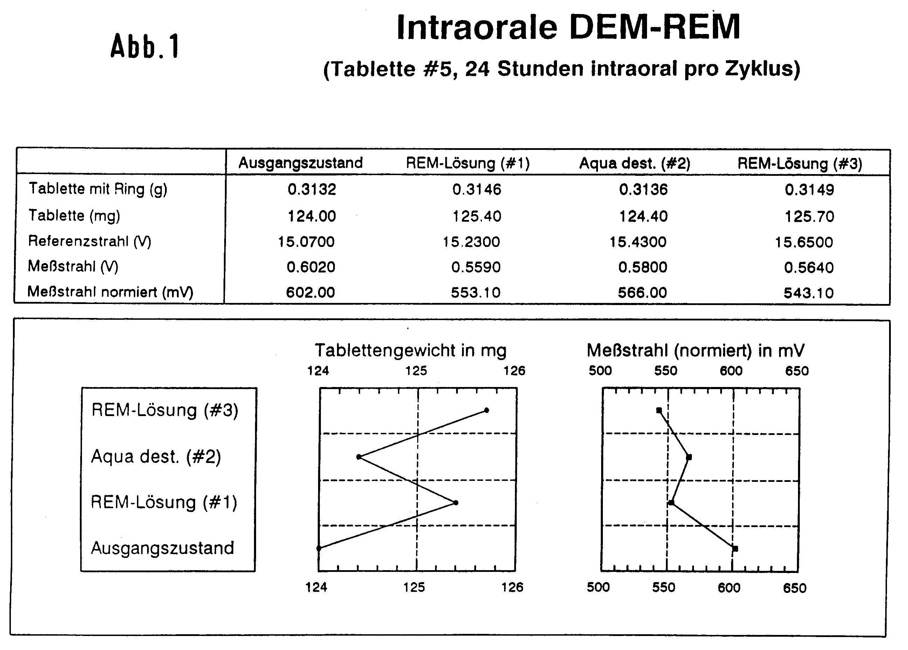

Abbildung 1 zeigt eine Messung des Massen(= Mineral)-zuwachses in einer porösen Hydroxylapatit-Probe. Solche Proben verhalten sich bezüglich Demineralisation und Remineralisation wie Zahnschmelz, der bekanntermaßen überwiegend aus diesem Material besteht. Die Proben wurden an einer Prothese im Mund getragen und dreimal täglich während der Mundpflege (5 min) in eine an Calzium und Phosphat gesättigten Lösung mit pH=4,5 eingebracht.

Als Kontrolle wurde in einem zweiten Durchgang die gleiche Probe jeweils in aqua dest. gelegt und in einer dritte Versuchsrunde wieder in die saure Ca-Phosphat-Lösung. Die Punkte des Diagramms zeigen jeweils das Gewicht der Probe zu Versuchsbeginn, nach dem ersten Tag mit saurer Ca-Phosphat-Lösung, nach einem Tag mit aqua dest. und nach wieder einem Tag mit saurer Ca-Phosphat-Lösung.

Deutlich ist ein Mineralzuwachs von jeweils 1,4 mg nach den beiden Versuchsdurchgängen mit Remineralisationslösung zu verzeichnen, während der Versuchsdurchgang mit aqua dest. einen Mineralverlust von 1 mg zur Folge hatte.

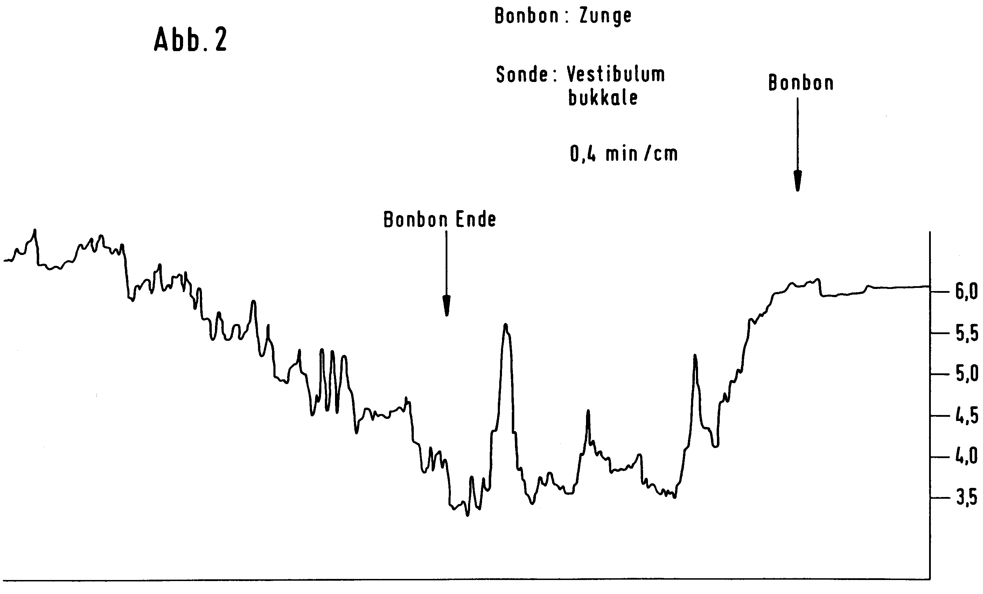

Beim Lutschen eines Bonbons mit z.B. 0,44 mol/kg Ca und 0,27 mol/kg PO4 ergibt sich bei einem Säuregehalt von 3,5 % Zitronensäure in der Wangentasche das in Abbildung 2 dargestellte zeitliche pH-Profil. Nach Einführen des Bonbons in die Mundhöhle sinkt der pH-Wert im Speichel innerhalb von 1 min auf pH 4 ab. Aufgrund der quaderförmigen Gestalt des Bonbons ist die Auflösung seiner Oberfläche zeitlich im wesentlichen konstant, so daß sich pro Zeit stets die gleiche Menge an Säure ablöst. Dadurch entsteht ein Plateau bei etwa pH 4. Nach Auflösung bzw. Entfernen des Bonbons steigt der pH-Wert im Speichel des Backenzahnbereiches durch die Speichelclearance wieder innerhalb von 3,2 min an. Entsprechende Kurven mit höherem pH-Plateau können mit anderen Säuregehalten erzielt werden.

## Patentansprüche

1. Präparat zur prophylaktischen und therapeutischen Behandlung von Karies in Form einer Kaumasse, von Kaubonbons, Lutschbonbons, Zahnpasta, Mundspray oder anderen Mundpflegemitteln, mit Calzium und Phosphat, die in gelöster oder leicht löslicher Form vorliegen, sowie einem Protonenspender, **dadurch gekennzeichnet**, daß
- spätestens bei der Anwendung in der Mundhöhle Calzium und Phosphat in eine gesättigte Lösung übergehen
- und der Säureanteil derart gewählt ist, daß sich der pH-Wert auf einen Wert zwischen 4 und 4,5 einstellt.

2. Präparat nach Anspruch 1, **dadurch gekennzeichnet**, daß als Grundmasse Zucker oder Zuckeraustauschstoffe und/oder Gelatine und/oder Gummiarabikum und/oder Kaumasse enthalten ist.

3. Präparat nach Anspruch 1 oder 2, **dadurch gekennzeichnet**, daß der Protonenspender eine organische Säure ist, insbesondere eine zur Aromatisierung von Süßwaren verwendete Säure, wie Zitronensäure, Milchsäure, Fruchtsäure, Weinsäure oder deren Mischungen.

4. Präparat nach einem der Ansprüche 1 bis 3, **dadurch gekennzeichnet**, daß die Calziumverbindung ein Calzium-Fruchtsäuresalz ist.

5. Präparat nach einem der Ansprüche 1 bis 4, **dadurch gekennzeichnet**, daß im festen Zustand der Anteil an Calzium 0,9 bis 3,6 Gewichtsprozent, vorzugsweise 1,35 bis 2,7 Gewichtsprozent, insbesondere 1,8 bis 2,25 Gewichtsprozent beträgt und der des Phosphates 0,47 bis 3,73, insbesondere 0,94 bis 2,82 beträgt sowie eine Menge von 400 bis 1500 mval Fruchtsäure pro Kilogramm Festmasse.

6. Präparat nach einem der Ansprüche 1 bis 5, **dadurch gekennzeichnet**, daß bei einer Zahnpasta ein Anteil von 160 bis 660 mmol Calziumverbindungen pro Kilogramm vorzugsweise 240 bis 500, insbesondere 320 bis 400 mmol/kg vorliegen und bei Phosphat ein Anteil von 40 bis 330 mmol/kg vorzugsweise 80 bis 250 mmol/kg und der Säureanteil 300 bis 1200 mval/kg Zahnpaste beträgt.

7. Präparat nach einem der Ansprüche 1 bis 6, **dadurch gekennzeichnet**, daß bei Mundwasser der Anteil des Calziums 5 bis 22 mmol insbesondere 10 bis 15 mmol beträgt und der Anteil des Phosphates 1 bis 10, vorzugsweise 3 bis 8 mmol und der Anteil der Säure 10 bis 40 mval/kg beträgt.

8. Präparat nach einem der Ansprüche 1 bis 7, **gekennzeichnet durch** einen Gehalt an einen die fraktale Abscheidung inhibierenden Stoff, insbesondere Fluorid in einer Konzentration von 0,7 bis 2,4 mmol/kg.

9. Präparat nach einem der Ansprüche 1 bis 8, **dadurch gekennzeichnet**, daß eine Calzium und/oder Phosphat adsorbierende Substanz beigegeben ist.

10. Präparat nach Anspruch 9, **dadurch gekennzeichnet**, daß die adsorbierende Substanz ein Polyglucan ist.

11. Präparat nach Anspruch 9, **dadurch gekennzeichnet**, daß die adsorbierende Substanz ein Muzin ist.

12. Präparat nach Anspruch 9, **dadurch gekennzeichnet**, daß die adsorbierende Substanz bipolar ist.

## Claims

1. Preparation for the prophylactic and therapeutic treatment of caries, in the form of a chewable mass, chewable sweets, sucking sweets, toothpaste, mouth wash, mouth spray or other oral hygiene means, having calcium and phosphate, which are present in a dissolved or freely soluble form, as well as a proton donor **wherein** that
- upon application in the oral cavity at the latest calcium and phosphate change into a saturated solution
- and the acidic proportion is selected in such a way that the pH value obtains a valve between 4 and 4.5.

2. Preparation according to claim 1, **wherein** as a base mass sugar or sugar substitute and/or gelatine and/or gum arabic and/or chewing mass is contained.

3. Preparation according to claim 1 or 2, **wherein** said proton donor is an organic acid, especially an acid used to aromatise confectionery, such as citric acid, lactic acid, fruit acid, tartaric acid or their mixtures.

4. Preparation according to one of claims 1 to 3, **wherein** the calcium compound is a calcium fruit acid salt.

5. Preparation according to one of claims 1 to 4, **wherein** in a solid state the proportion of calcium is 0.9 to 3.6 weight per cent, preferably 1.35 to 2.7 weight per cent, especially 1.8 to 2.25 weight per cent and that of the phosphate is 0.47 to 3.73, especially 0.94 to 2.82 and a quantity of 400 to 1500 millival of fruit acid per kilogram of solid mass.

6. Preparation according to one of claims 1 to 5, **wherein** in the case of a toothpaste there is a proportion of 160 to 660 mM of calcium compounds per kilogram preferably 240 to 500, especially 320 to 400 mM/kg and in the case of phosphate a proportion of 40 to 330 mM/kg preferably 80 to 250 mM/kg and the acid proportion is 300 to 1200 millival/kg of toothpaste.

7. Preparation according to one of claims 1 to 6, **wherein** in the case of mouth wash the proportion of calcium is 5 to 22 mM, especially 10 to 15 mM and the proportion of the phosphate is 1 to 10, preferably 3 to 8 mM and the proportion of the acid is 10 to 40 millival/kg.

8. Preparation according to one of claims 1 to 7, **wherein** the content of a substance that inhibits fractional precipitation, especially fluoride is in a concentration of 0.7 to 2.4 mM/kg.

9. Preparation according to one of claims 1 to 8, **wherein** a calcium and/or phosphate adsorbing substrate is added.

10. Preparation according to claim 9, **wherein** the adsorbing substance is a polyglucan.

11. Preparation according to claim 9, **wherein** the adsorbing substance is a mucin.

12. Preparation according to claim 9, **wherein** the adsorbing substance is bipolar.

## Revendications

1. Préparation destinée au traitement prophylactique et thérapeutique de la carie dentaire sous forme de pâte à mâcher, de bonbons à mâcher, de bonbons à sucer, de pâte dentifrice, d'aérosol buccal ou d'autres produits servant à l'hygiène buccale composée de calcium et de phosphate dissous ou présents sous une forme facilement soluble ainsi que d'un dispensateur de protons **caractérisée en ce que**
- au plus tard pendant l'utilisation dans la cavité buccale, le calcium et le phosphate se dissolvent et se transforment en une solution saturée
- et que l'acidité est choisie de telle façon que le pH est situé dans une plage comprise entre un pH de 4 et un pH de 4,5.

2. Préparation selon la revendication 1, **caractérisée par le fait que** la pâte de base contient du sucre ou des substituts du sucre et/ou de la gélatine et/ou de la gomme arabique et/ou une pâte à mâcher.

3. Préparation selon la revendication 1 ou 2, **caractérisée par le fait que** le dispensateur de protons est un acide organique, en particulier un acide utilisé pour aromatiser les sucreries, comme l'acide citrique, l'acide lactique, un acide de fruits, l'acide tartrique ou un mélange de ces différents acides.

4. Préparation selon une des revendications 1 à 3, **caractérisée en ce que** la combinaison de calcium est un sel calcique d'acide de fruits.

5. Préparation selon une des revendications 1 à 4 **caractérisée par le fait qu'à** l'état solide, la proportion de calcium est de 0,9 à 3,5 % en poids, elle atteint de préférence 1,37 à 2,7% en poids et notamment 1,8 à 2,25% en poids, que le pourcentage pondéral du phosphate est de 0,47 à 3,73% notamment de 0,94 à 2,82% et que la quantité d'acide de fruits est de 400 à 1500 mval par kilogramme de pâte solide.

6. Préparation selon une des revendications 1 à 5 **caractérisée en ce que**, pour une pâte dentifrice, la teneur en combinaisons de calcium est de 160 à 660 mmoles par kilogramme, de préférence de 240 à 500, notamment de 320 à 400 mmoles/kg, la teneur en phosphate étant de 40 à 330 mmoles/kg, de préférence 80 à 250 mmoles/kg et que la teneur en acide atteint 300 à 1200 mval/kg de pâte dentifrice.

7. Préparation selon une des revendications 1 à 6 **caractérisée en ce que**, pour une eau de bouche, la teneur en calcium est de 5 à 22 mmoles, notamment de 10 à 15 mmoles et que la teneur en phosphate est de 1 à 10 mmoles, de préférence de 3 à 8 mmoles et que la teneur en acide est de 10 à 40 mval/kg.

8. Préparation selon une des revendications 1 à 7 **caractérisée en ce qu'une** substance inhibant le dépôt fragmenté notamment du fluorure est présent dans une concentration de 0,7 à 2,4 mmoles/kg.

9. Préparation selon une des revendications 1 à 8 **caractérisée par** l'adjonction d'une substance adsorbant le calcium et/ou le phosphate.

10. Préparation selon la revendication 9 **caractérisée par le fait que** la substance adsorbante est un polyglucane.

11. Préparation selon la revendication 9, **caractérisée par le fait que** la substance absorbante est une mucine.

12. Préparation selon la revendication 9, **caractérisée par le fait que** la substance adsorbante est bipolaire.
